# EUROPÄISCHE PATENTSCHRIFT

(11) **EP 2 066 314 B1**
(45) Veröffentlichungstag und Bekanntmachung des Hinweises auf die Patenterteilung: **04.12.2013**
(21) Anmeldenummer: 07818393.6
(22) Anmeldetag: 25.09.2007
(51) Int. Cl.: A61P 25/00, A61K 31/235

(54) **VERWENDUNG VON THEOGALLIN ZUR BEHANDLUNG VON MENTALEN KONZENTRATIONSLEISTUNGSSTÖRUNGEN, DEPRESSION UND DEMENZ**
USE OF THEOGALLIN FOR THE TREATMENT OF MENTAL CONCENTRATION DISORDERS, DEPRESSION AND DEMENTIA
UTILISATION DE THÉOGALLINE DANS LE TRAITEMENT DE TROUBLES DE LA CONCENTRATION MENTALE, LA DÉPRESSION ET LA DÉMENCE

(30) Priorität: 26.09.2006 DE 102006045764
(43) Veröffentlichungstag der Anmeldung: 10.06.2009
(73) Patentinhaber: Plantextrakt GmbH & Co. KG, 91487 Vestenbergsgreuth (DE)
(72) Erfinder: KLER, Adolf, Dr., 91413 Neustadt/Aisch (DE); ZENGER, Reinhold, Dr., 91325 Adelsdorf (DE); DIMPFEL, Wilfried, Prof. Dr., 35578 Wetzlar (DE)
(74) Vertreter: Hübner, Gerd
(86) Internationale Anmeldenummer: PCT/EP2007/008308
(87) Internationale Veröffentlichungsnummer: WO 2008/037416

(56) Entgegenhaltungen:
- WO-A-2007/079331
- HASEGAWA T: "PREVENTIVE EFFECT OF GREEN TEA AGAINST ALZHYMEIR-TYPE AND VASCULAR TYPE SENILE DEMENTIA" NEUROSCIENCE RESEARCH SUPPLEMENT, ELSEVIER, SHANNON, IR, Nr. 24, 2000, Seite S28, XP001196939 ISSN: 0921-8696
- KUHR S ET AL: "BESTIMMUNG VOM FLAVANOLEN, THEOGALLIN, GALLUSSAEURE UND COFFEIN IN TEE MITTELS HPLC DETERMINATION OF FLAVANOLS THEOGALLIN GALLIC ACID AND CAFFEINE IN TEA USING HLPC" ZEITSCHRIFT FUER LEBENSMITTELUNTERSUCHUNG UND -FORSCHUNG, XX, XX, Bd. 192, Nr. 6, 1991, Seiten 526-529, XP009094190 ISSN: 0044-3026
- JUKKA HINTIKKA ET AL: "Daily tea drinking is associated with a low level of depressive symptoms in the Finnish general population" EUROPEAN JOURNAL OF EPIDEMIOLOGY, KLUWER ACADEMIC PUBLISHERS, DO, Bd. 20, Nr. 4, 1. April 2005 (2005-04-01), Seiten 359-363, XP019239074 ISSN: 1573-7284
- CHATURVEDI ET AL: "Neuroprotective and neurorescue effect of black tea extract in 6-hydroxydopamine-lesioned rat model of Parkinson's disease" NEUROBIOLOGY OF DISEASE, BLACKWELL SCIENCE, OXFORD, GB, Bd. 22, Nr. 2, Mai 2006 (2006-05), Seiten 421-434, XP005404631 ISSN: 0969-9961

## Beschreibung

Die vorliegende Erfindung betrifft die Verwendung von Theogallin, seines Metaboliten Chinasäure oder deren pharmazeutisch annehmbaren Salzen zur Herstellung eines Arzneimittels zur Prophylaxe und Behandlung von mentalen Konzentrationsleistungsstörungen, Depression und Demenz.

Theogallin ist in einer Reihe von Naturprodukten enthalten, insbesondere in Grünteeextrakten. Es hat folgende Summenformel:

C₁₄H₁₆O₁₀

Chinasäure hat folgende Summenformel:

C₇H₁₂O₆

Eine biologische oder pharmakologische Wirkung wurde bislang noch nicht beschrieben.

Die Fachveröffentlichung HASEGAWA T: "PREVENTIVE EFFECT OF GREEN TEA AGAINST ALZHYMEIR-TYPE AND VASCULAR TYPE SENILE DEMENTIA" NEUROSCIENCE RESEARCH SUPPLEMENT, ELSEVIER, SHANNON, IR, Nr. 24, 2000, Seite S28, XP001196939 ISSN: 0921-8696 beschäftigt sich mit einem vorbeugenden Effekt von Grüntee gegen Altersdemenz auf Grund von Alzheimer- oder Gefäßerkrankungen. Es wird auf den protektiven Effekt von grünem Tee an sich hingewiesen; eine Korrelation, welche Substanzen im Grüntee für diese protektive Wirkung verantwortlich sind, wird an keiner Stelle dieses Dokumentes offenbart. Es wird lediglich davon gesprochen, dass eine tägliche Polyphenol-Aufnahme von 0,3 bis 1,8 g notwendig sei, um ältere Personen vor einer Abnahme der kognitiven Leistung zu schützen. Auf eine pharmakologische Wirkung von Theogallin bzw. seines Metaboliten Chinasäure gibt dieses Dokument keinerlei Hinweis.

Die Fachveröffentlichung JUKKA HINTIKKA ET AL: "Daily tea drinking is associated with a low level of depressive symptoms in the Finnish general population" EUROPEAN JOURNAL OF EPIDEMIOLOGY, KLUWER ACADEMIC PUBLISHERS, DO, Bd. 20, Nr. 4, 1. April 2005 (2005-04-01), Seiten 359-363, XP019239074 ISSN: 1573-7284 beschäftigt sich mit einer Untersuchung über die antidepressive Wirkung des täglichen Konsums von Tee. Dabei wird in erster Linie auf den Bestandteil Koffein und dessen - je nach Dosierung - euphorisierende bzw. depressionsfördernde Wirkung hingewiesen (siehe a.a.O., Abschnitt "Introduction", Abs. 1). Ferner werden Catechine und Spurenelemente wie Chrom, Mangan, Selen und Zink als Bestandteile von Tee erwähnt, wobei den Catechinen eine neuroprotektive Aktivität als positive Eigenschaft zugesprochen wird (siehe a.a.O., Abschnitt "Introduction", Abs. 2, letzter Satz). Auf die Stoffe Theogallin bzw. Chinasäure und deren in der Patentanmeldung beschriebenen Wirkungen erfolgt kein Hinweis.

Die Fachveröffentlichung CHATURVEDI ET AL: "Neuroprotective and neurorescue effect of black tea extract in 6-hydroxydopamine-lesioned rat model ofParkinson's disease" NEUROBIOLOGY OF DISEASE, BLACKWELL SCIENCE, OXFORD, GB, Bd. 22, Nr. 2, Mai 2006 (2006-05), Seiten 421-434, XP005404631 ISSN: 0969-9961 beschäftigt sich mit der neurologischen Wirkung von Schwarzteeextrakt und stellt einen umfassenden Tierversuch mit Ratten vor, denen wässriger Schwarzteeextrakt über eine bestimmte Zeit hinweg verabreicht wurde. Die neurologische Wirkung von Grüntee, Schwarztee und ihrer Bestandteile wird lediglich oberflächlich in der Einleitung angesprochen (siehe a.a.O., Seite 422, linke Spalte, Abs. 3). Genannt werden lediglich Catechine, Theaflavine, Thearubigene und Flavonoide. Zu Theogallin bzw.Chinasäure erfolgt keine Aussage.

Schließlich ist auf die Fachveröffentlichung KUHR S ET AL: "BESTIMMUNG VOM FLAVANOLEN, THEOGALLIN, GALLUSSAEURE UND COFFEIN IN TEE MITTELS HPLC - DETERMINATION OF FLAVANOLS THEOGALLIN GALLIC ACID AND CAFFEINE IN TEA USING HLPC" ZEITSCHRIFT FUER LEBENSMITTELUNTERSUCHUNG UND -FORSCHUNG, XX, XX, Bd. 192, Nr. 6, 1991, Seiten 526-529, XP009094190 ISSN: 0044-3026 zu verweisen, das die Analyse verschiedenster Teesorten mit Hilfe der Hochleistungsflüssigkeitschromatographie (HPLC) offenbart. Daraus wird deutlich, dass Theogallin ein Teebestandteil ist. Wie aus den beiden Tabellen 1 und 2 in diesem Dokument hervorgeht, ist Theogallin ein mengenmäßig untergeordneter Bestandteil.

Die derzeit üblichen Arzneimittel zur Behandlung von Krankheiten des zentralen Nervensystems, insbesondere von Demenz, Depression und Konzentrationsleistungsstörungen haben ein breites Nebenwirkungsspektrum. Es besteht daher ein sehr großer Bedarf nach einer Prophylaxe bzw. einem verbesserten Arzneimittel mit einer guten therapeutischen Wirksamkeit bei möglichst geringer Nebenwirkungsrate. Insbesondere gilt dies auch für die Prophylaxe.

Der Erfindung liegt insoweit die Aufgabe zugrunde, diesen Bedarf zu decken.

Diese Aufgabe wird gemäß Anspruch 1 überraschenderweise durch die Verwendung des Naturstoffs Theogallin sowie dessen im Körper entstehender Metabolit Chinasäure oder von deren pharmazeutisch annehmbaren Salzen als Wirkstoff für die Herstellung eines Arzneimittels zur Prophylaxe und Behandlung von Demenzen Depressionen oder Konzentrationsleistungsstörungen, gelöst. Erwähnenswert dabei ist die therapeutische Wirkung bezüglich des Aufmerksamkeitsdefizit-Hyperaktivitätssyndroms bei Kindern.

Auf der Basis der bisher bekannten Literatur war eine Wirkung von Theogallin bei pathologischen Störungen des zentralen Nervensystems, insbesondere von neurologischen und psychiatrischen Erkrankungen sowie Konzentrationsleistungsstörungen nicht zu erwarten gewesen. Erst die Anwendung der völlig "bias"-freien Untersuchung im Tele-Stereo-EEG der Ratte erbrachte im Rahmen von Versuchen mit anderer Zielsetzung den Hinweis auf das Vorhandensein einer derartigen zentralnervösen Wirkung. Die Wirkung wurde weiterhin durch in vitro Versuche am Hippokampus-Schnittpräparat in direkter Interaktion zwischen Wirkstoff und seinem Zielorgan, dem Gehirn, untermauert.

Bevorzugte Ausführungsformen der Erfindung sowie weitere Merkmale, Einzelheiten und Vorteile ergeben sich aus den abhängigen Ansprüchen und der nachfolgenden Beschreibung, die auf die beigefügten Diagramme Bezug nimmt. Es zeigen:
- **Fig. 1**: ein Diagramm zur Darstellung der Wirkung von Theogallin und Chinasäure auf EEG Frequenzen während der ersten Stunde nach dessen Applikation (Mittelwerte von n=4 Tieren),
- **Fig. 2**: ein Diagramm zur Darstellung der Wirkung verschiedener Substanzen wie Haloperidol (Neuroleptika), Paroxetin (Antidepressiva, Schmerzen), Amphetamin (Stimulants), Diazepam (Sedativa) und Valproinsäure (Antikonvulsiva),
- **Fig: 3**: ein Diagramm der konzentrationsabhängigen Zunahme der Amplitude des Populationsspikes (Aktivität der Pyramidenzellen) in Gegenwart von Theogallin im Hippokampus Schnittpräparat in vitro (Mittelwerte von je 4 Schnitten mit mittlerem Fehler des Mittelwertes);
- **Fig. 4**: ein Diagramm des Amplitudenverhaltens des Populationsspikes im Hippokampus Schnittpräparat nach Theta-Burst-Stimulation in Gegenwart von Theogallin;
- **Fig. 5**: ein Diagramm der konzentrationsabhängigen Zunahme der Amplitude des Populationsspikes (Aktivität der Pyramidenzellen) in Gegenwart von Chinasäure im Hippokampus Schnittpräparat in vitro (Mittelwerte von je 4 Schnitten mit mittlerem Fehler des Mittelwertes); und
- **Fig. 6**: ein Diagramm des Amplitudenverhaltens des Populationsspikes im Hippokampus Schnittpräparat nach Theta-Burst-Stimulation in Gegenwart von Chinasäure.

Die Erfindung bezieht sich auf die Verwendung von Theogallin bzw. seinem Metaboliten Chinasäure zur Herstellung eines Medikamentes zur Prophylaxe und Behandlung von Demenzen, Depressionen und Konzentrationsleistungsstörungen. Theogallin hat folgende Summenformel:

C₁₄H₁₆O₁₀

Erfindungsgemäß wird neben Theogallin auch sein Metabolit Chinasäure eingesetzt. Chinasäure hat folgende Summenformel:

C₇H₁₂O₆

Theogallin oder Chinasäure kann erfindungsgemäß auch in Form seiner pharmazeutisch annehmbaren Salze oder Komplexe eingesetzt werden. Die Herstellung solcher Salze erfolgt in an sich bekannter Weise. Als Salzbildner kommen alle üblichen pharmazeutisch annehmbaren Säuren bzw. Anionen in Frage. Auch eine Kopplung an Glucuronsäure kann verwendet werden. Insbesondere kann auch Chinasäure allein verwendet werden.

Die erfindungsgemäßen Theogallin oder Chinasäure als Wirkstoff enthaltenden Arzneimittel werden bevorzugt oral verabreicht. Es ist jedoch auch eine Verabreichung auf anderem Wege, z.B. peroral, topisch, parenteral, intravenös, intramuskulär, subkutan, nasal, inhalativ, rektal oder transdermal möglich.

Zur Verabreichung kann das erfindungsgemäße Arzneimittel, das als Wirkstoff Theogallin oder Chinasäure enthält, z.B. in Form von Pulvern, Tabletten, Kapseln, Pillen, Dragees, Granulaten, Suppositorien, Pellets, Sirupe, Lösungen oder Dispersionen formuliert werden, wobei der Wirkstoff optional mit pharmazeutisch annehmbaren Hilfs- und Trägerstoffen kombiniert werden kann.

Liegt das erfindungsgemäße Arzneimittel in Form einer Lösung vor, so enthält diese bevorzugt 0.1 bis 10 Gew.-%, besonders bevorzugt 1 bis 5 Gew.-% des Wirkstoffs.

Die erfindungsgemäßen Arzneimittel, die Theogallin oder Chinasäure als Wirkstoff enthalten, werden normalerweise gemäss herkömmlichen Methoden hergestellt und in einer pharmazeutisch geeigneten Form verabreicht.

Zum Beispiel können die festen oralen Formen zusammen mit dem Wirkstoff Streckstoffe (z.B. Lactose, Dextrose, Saccharose, Cellulose, Maisstärke oder Kartoffelstärke), Gleitmittel (z.B. Silikat, Talk, Stearinsäure, Magnesium- oder Calciumstearat und/oder Polyethylenglykole), Bindemittel (z.B. Stärken, Gummi arabicum, Gelatine, Methylcellulose, Carboxymethylcellulose oder Polyvinylpyrrolidon), Aufschussmittel (z.B. Stärke, Alginsäure, Alginate oder Natriumstärkeglykolate), aufschäumende Mischungen, Farbstoffe, Süßungsmittel Benetzungsmittel (z.B. Lecithin, Polysorbate, Laurylsulfate) und im allgemeinen nicht-toxische und pharmakologisch inaktive Substanzen, die in pharmazeutischen Formulierungen verwendet werden, enthalten.

Die pharmazeutischen Zubereitungen können in bekannter Weise hergestellt werden, z.B. mittels Mischen, Granulieren, Tablettieren, Zuckerbeschichtungs- oder Überzugsbeschichtungsverfahren.

Die flüssigen Dispersionen zur oralen Verabreichung können z.B. Sirupe, Emulsionen und Suspensionen sein.

Sirupe können als Träger z.B. Saccharose oder Saccharose mit Glycerin und/oder Mannitol und/oder Sorbitol enthalten.

Die Suspensionen und die Emulsionen können als Träger z.B. ein natürliches Harz, Agar, Natriumalginat, Pectin, Methylcellulose, Carboxymethylcellulose oder Polyvinylalkohol enthalten.

Die Suspensionen oder Lösungen für intramuskuläre Injektionen können zusammen mit dem Wirkstoff einen pharmazeutisch annehmbaren Träger, z.B. steriles Wasser, Olivenöl, Ethyloleat, Glykole, z.B. Propylenglykol, und, falls gewünscht, eine geeignete Menge an Lidocain-Hydrochlorid enthalten.

Die Lösungen zur intravenösen Injektion oder Infusion können als Träger z.B. steriles Wasser enthalten oder sie können bevorzugt in Form von sterilen, wässrigen, isotonsichen Salzlösungen vorliegen.

Die Suppositorien können zusammen mit dem Wirkstoff einen pharmazeutisch annehmbaren Träger, z.B. Kakaobutter, Polyethylenglykol, einen Polyoxyethylensorbitol-Fettsäureester oder Lecithin enthalten.

Zusammensetzungen für die topische Applikation, z.B. Cremes, Lotionen oder Pasten, können durch Mischen des Wirkstoffs mit einem herkömmlichen ölhaltigen oder emulgierenden Träger hergestellt werden.

Die Dosierungseinheit des Arzneimittels kann beispielsweise enthalten:
- bei peroralen Arzneiformen bevorzugt 80 bis 200 mg, besonders bevorzugt 90 bis 120 mg, Wirkstoff pro Tagesdosis - die Tagesdosis kann beispielsweise in 1 bis 3 Einzeldosen, vorzugsweise in zwei Einzeldosen, täglich verabreicht werden.
- bei parenteralen Arzneiformen (zum Beispiel intravenös, subkutan, intramuskulär) bevorzugt 20 bis 60 mg, besonders bevorzugt 30 mg, Wirkstoff pro Tagesdosis - die Tagesdosis kann beispielsweise in 1 bis 3 Einzeldosen, vorzugsweise in einer Einzeldosis, täglich verabreicht werden.
- bei Arzneiformen zur rektalen Applikation bevorzugt 40 bis 80 mg, besonders bevorzugt 60 mg, Wirkstoff pro Tagesdosis - die Tagesdosis kann beispielsweise in 1 bis 3 Einzeldosen, vorzugsweise in einer Einzeldosis, täglich verabreicht werden.
- bei Arzneiformen zur Applikation auf die Haut und Schleimhäute (z.B. Lösungen, Lotionen, Emulsionen, Salben usw.) bevorzugt 40 bis 80 mg Wirkstoff, besonders bevorzugt 60 mg, Wirkstoff pro Einzeldosis - die Tagesdosis kann beispielsweise in 1 bis 6 Einzeldosen, vorzugsweise in 1 -3 Einzeldosen, täglich verabreicht werden.

Bei Verwendung eines pharmazeutisch annehmbaren Salzes bzw. Derivates muss die verwendete Menge in dem Fachmann bekannter Weise entsprechend angepaßt werden.

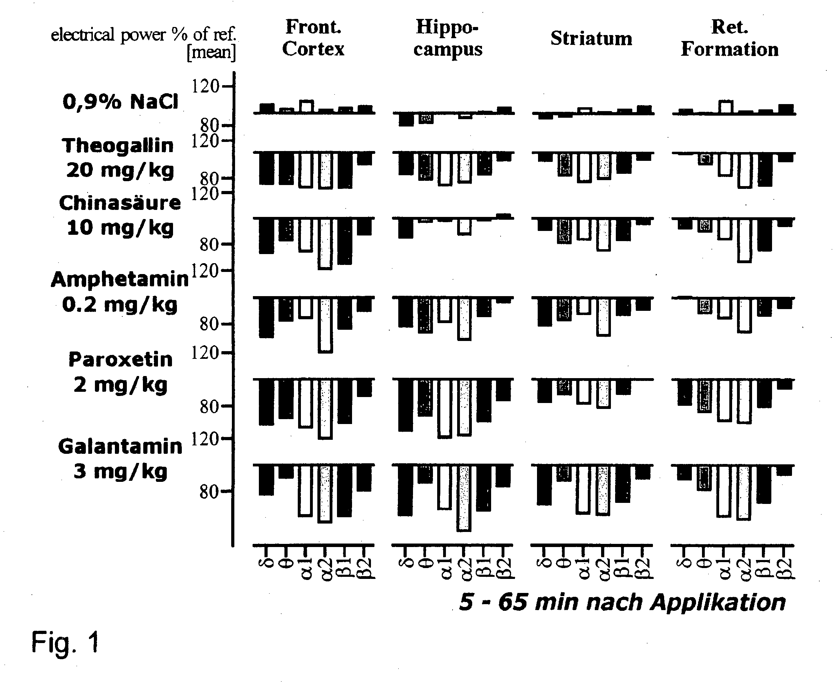

In vitro wie auch in vivo Versuche an der Ratte zeigen Ergebnisse, wie sie für Medikamente zur Behandlung von Depression, Demenzen und Konzentrationsleistungsstörungen typisch sind. Eine Übersicht ist in Fig. 1 gegeben.

Eine erste Verabreichung eines Tee-Extraktes an Probanden, der diese Substanzen enthält, zeigte die vermutete Wirksamkeit auch am Menschen. Bei Untersuchungen der Veränderungen der EEG-Frequenzen der Ratte nach Gabe von Theogallin und Chinasäure sowie der direkten Interaktion von Theogallin und Chinasäure mit Hirnmaterie im Hippokapusschnittpräparat der Ratte in vitro wurde überraschenderweise gefunden, dass das Präparat eine Wirkung im Gehirn besitzt, die bei der Behandlung von neurologischen und psychiatrischen Erkrankungen des Gehirns, insbesondere von Demenz, Depression und Konzentrationsleistungsstörungen nützlich ist.

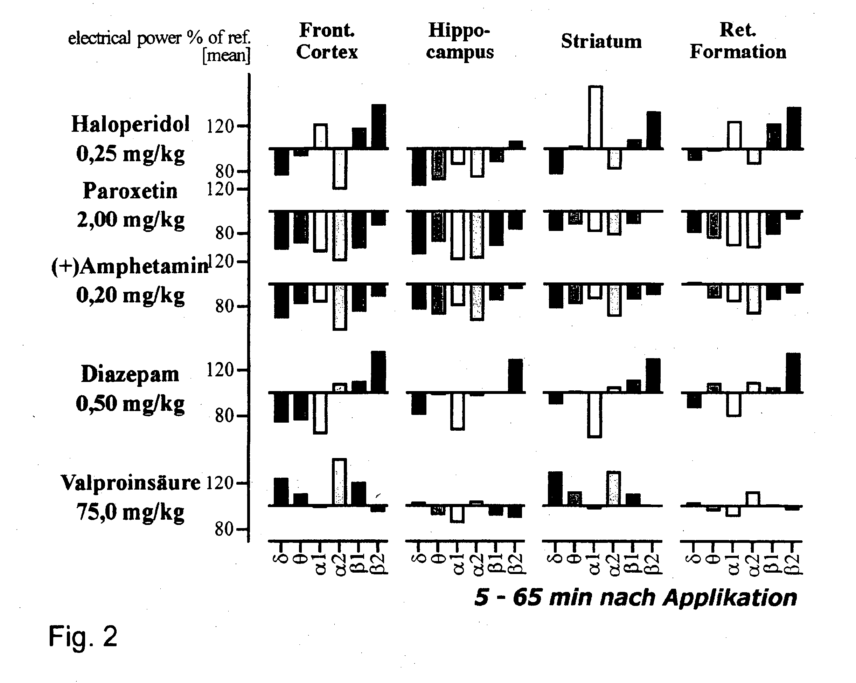

Theogallin zeigt überraschenderweise im Modell Tele-Stereo-EEG bei Ratten Veränderungen der EEG-Frequenzen, wie sie nach Gabe klassischer Medikamente zur Behandlung von Demenzen (Beispiel Galanthamin), Depression (Beispiel Paroxetin) sowie mentalen Leistungsstörungen (Beispiel Amphetamin) gemessen werden. Die Veränderungen, wie sie nach Gabe klassischer Medikamente sowie nach Gabe von einer Dosis von Theogallin (20 mg/kg) auftraten, sind in Fig. 1 gezeigt. Aus der in Fig. 2 gezeigten Abbildung ist offensichtlich, dass sich der charakteristische elektrische Fingerprint, der für klassische Medikamente zur Behandlung von Demenzen, Depression und Konzentrationsleistungsstörungen typisch ist, auch in den Mustern von Theogallin und Chinasäure in signifikanter Weise wiederfindet. Deutlich erkennbar ist die Übereinstimmung bei den Substanzen Theogallin/Chinasäure einerseits und Amphetamin/Paroxetin andrerseits.

Die Fig. 1 zeigt die zeitabhängigen Veränderungen der EEG-Frequenzen nach Gabe einer Dosis von Theogallin wie sie gemäß dem nachfolgend erläuterten Testverfahren 1 gemessen wurden. Aus der Erkenntnis, dass Theogallin und Chinasäure die gleichen charakteristischen Veränderungen der EEG-Frequenzen hervorruft wie üblicherweise Arzneimittel, die zur Behandlung von Demenzen, Depressionen und Konzentrationsleistungsstörungen eingesetzt werden, kann gefolgert werden, dass Theogallin bzw. Chinasäure bei der Behandlung in denselben Indikationen wirksam ist. Dass Medikamente, die in der gleichen Indikation eingesetzt werden, auch gleichartige EEG Veränderungen hervorrufen, konnte von Dimpfel anhand von mehr als 40 Referenzsubstanzen für 8 verschiedene Indikationen gezeigt werden (Dimpfel W.: "Preclinical data base of pharmaco-specific rat EEG fingerprints (Tele-Stereo-EEG)" in Eur J Med Res (2003) 8: 199-207).

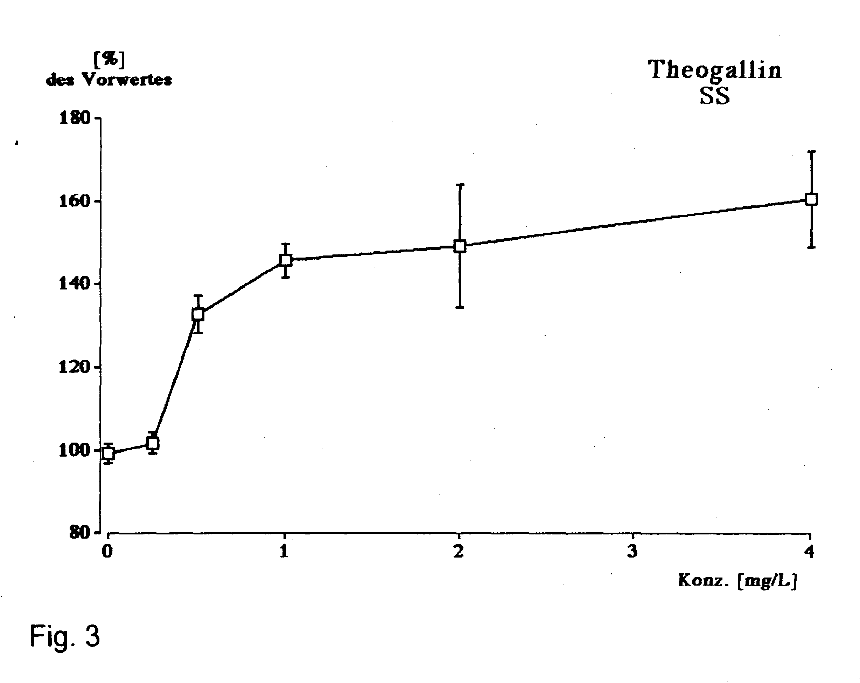

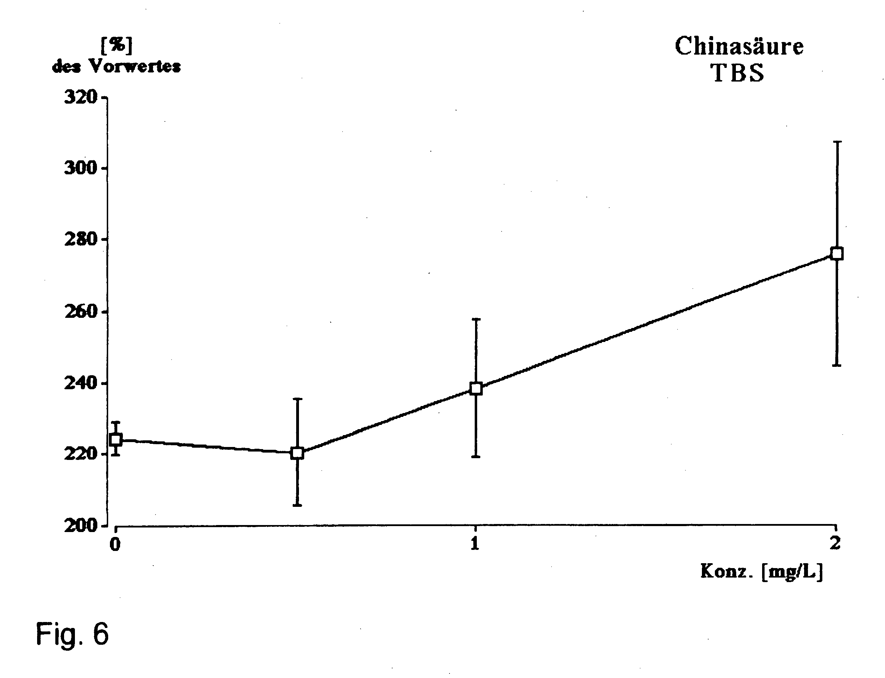

Zusätzlich wurden Untersuchungen am Modell Hippokampus-Schnittpräparat in vitro durchgeführt. In diesen Untersuchungen wurde überraschend gefunden, dass Theogallin und Chinasäure sowohl eine Zunahme der Pyramidenzellaktivität nach Einzelreizung wie auch eine Zunahme der Langzeitpotenzierung hervorruft (siehe Fig. 3 bis 6). Dieses Phänomen wurde für andere stimulierende und antidementiv wirkende Arzneimittel wie Memantine in der Literatur bereits berichtet (Dimpfel W.: "Effects of Memantine on synaptic transmission in the hippocampus in vitro" in Arzneim.-Forsch/Drug Res (1995) 45: 1-5).

### Testverfahren 1 (Tele-Stereo-EEG)

Die Veränderungen der EEG-Frequenzen wurde nach Gabe von Salzlösung (Kontrolle) bzw. oral von Theogallin (20 mg/kg Körpergewicht) sowie oral 10 mg/kg der Chinasäure bestimmt.

Die Untersuchungen wurden analog der durch W. Dimpfel beschriebenen Methode (Dimpfel W.: "Preclinical data base of pharmaco-specific rat EEG fingerprints (Tele-Stereo-EEG)" in Eur J Med Res (2003) 8: 199-207) folgendermaßen durchgeführt:

Vier männlichen erwachsenen Fischer-344 Ratten (Tag-Nacht konvertiert) wurden im Alter von 6 Monaten 4 bipolar konzentrische Elektroden zusammen mit einem Mikrostecker auf einer gemeinsamen Basisplatte implantiert. Der Stecker diente der Aufnahme eines 4-Kanal-Senders zur telemetrischen Übertragung der aus frontalem Kortex, Hippokampus, Striatum und Formatio Reticularis abgeleiteten Feldpotentiale. Die Signale wurden auf einem Computer System (Software "EEG-Analyse", Betriebssystem OS Science, Laborrechner "LabTeam" der Firma MediSyst, Linden, DE) in Echtzeit einer Fast-Fourier-Transformation unterworfen und die Leistungsdichtespektren jeweils über 60 Minuten gemittelt. Die Unterteilung der Spektren in 6 verschiedene Frequenzbereiche erlaubte die Erfassung pharmako-spezifischer Veränderungen in Bezug auf die jeweils vor Applikation gemessenen Vorwerte innerhalb dieser Frequenzbänder.

Zum Applikationsprotokoll der Substanzen ist festzuhalten, dass diese oral 45 Minuten nach Beginn der Messungen (Vorwert) appliziert wurden. Fünf Minuten später wurden die Messungen wieder gestartet, mindestens über die nächsten 5 Stunden kontinuierlich analysiert und in 60-minütigen Perioden zusammengefaßt. Die Testsubstanzen wurden in einer Dosierung von 20 mg/kg (Theogallin) bzw. 10 mg/kg (Chinasäure) appliziert. Die experimentelle Serie wurde mit der Injektion von Salzlösung (Kontrolle), die zu keiner auffälligen Änderung führte, begonnen.

Der statistische Vergleich der Versuche zu den Ergebnissen, die nach Gabe von Salzlösung gemessen wurde, erfolgte mit Hilfe einer multivarianten Analyse nach Ahrens und Läuter (siehe H. Ahrens, J. Läuter: "Mehrdimensionale Varianzanalyse" (1974), Akademie Verlag, Berlin) auf der Basis der Veränderungen innerhalb der einzelnen Frequenzbänder in allen Himregionen als Variablen.

Die Verabreichung von Salzlösung führte kaum zu Veränderungen der elektrischen Aktivität (µV²/Ω) im Vergleich zu den Vorphasenwerten.

Die Verabreichung von Theogallin führte zu deutlichen Veränderungen der Leistungsdichte im frontalen Kortex. (siehe Fig.1). Die Verabreichung von Chinasäure führte zu ähnlichen Ergebnissen, wobei die Veränderungen im frontalen Kortex sich von denen nach Gabe von Salzlösung mit einer Irrtumswahrscheinlichkeit von p<5% unterschieden.

Die orale Verabreichung von Theogallin als Einzeldosis führt zu Veränderungen der elektrischen Hirnaktivität bei den Testtieren, die denen nach Gabe von Paroxetine bzw. Galanthamin oder Amphetamin entsprach. Dieses Muster korreliert in auffälliger Weise mit den Mustern, die für Medikamente, wie sie bereits für die Behandlung von Demenzen und Depressionen üblicherweise verwendet werden (siehe Fig. 1), nicht jedoch mit Mustern, die bei Medikamenten für andere Indikationen auftreten. Bereits in der ersten Stunde wurden im frontalen Kortex Abnahmen der delta und alpha2 Aktivität beobachtet (siehe Fig. 1), wie sie typischerweise für die Applikation von Stimulantien, Antidepressiva oder Antidementiva zu diesem Zeitpunkt gemessen wurden.

Die beobachteten Frequenzänderungen wurden mittels einer Diskriminazanalyse mit den Ergebnissen von Medikamenten zur Behandlung von neurologischen und psychiatrischen Erkrankungen des Gehirns wie Demenzen, Depression wie auch mit Medikamenten für andere Indikationen verglichen. Folgende Arzneimittel, welche bereits im selben Modell geprüft wurden, standen für Vergleichszwecke zur Verfügung: Memantine 1.0 mg; Haloperidol 0.5 mg; Chlorpromazin 0.5 mg; Prothipendyl 1 mg; Clozapine 3 mg; Thioridazin 5 mg; Phentytoin 4 mg; Carbamazepin 15 mg; Valproinsäure 75 mg; Diazepam 0.5 mg; Methohexital 20 mg; Midazolam 0.5 mg; Meprobamat 60 mg; Phenobarbital 60 mg; Amphetamin 0.2 mg; Fenfluramin 1 mg; Kokain 10 mg; Mescalin 10 mg; Metanicotin 1mg; R-DOI 0.1 mg; R-DOM 0.2 mg; Dizocilpin 0.25 mg; LSD 0.05 mg; Acetylsalicylsäure 200 mg; Metamizol 100mg; Fentanyl 0.075 mg; Tramadol 10mg; Fluvoxamin 40 mg; Nomifensin 1mg; Mianserin 5 mg; Amitriptylin 10mg; Imipramin 10 mg; Paroxetin 2 mg; Galanthamin: 3 mg. Die Angaben beziehen sich jeweils auf eine Dosierung in mg/kg Körpergewicht. Abbildung 2 zeigt stellvertretend fünf dieser Substanzen.

### Testverfahren 2 (Langzeitpotenzierung im Hippokampus-Schnittpräparat in vitro)

Wirkungen von Theogallin und Chinasäure auf die Erregbarkeit der Pyramidenzellen wurden im Hippokampus-Schnittpräparat der Ratte bestimmt. Die Analyse der Wirkungen erfolgte auf zwei Ebenen. Zunächst wurde anhand der Applikation von Einzelreizen die normale Erregbarkeit überprüft und anschließend nach einer sog. "Theta-burst-Stimulation" die Auswirkungen auf die Langzeitpotenzierung gemessen.

Für die Durchführung dieser Studien wurden 9 erwachsene männliche CD-Ratten verwendet. Die Isolation des Hippokampus erfolgte an äthemarkotisierten und anschließend exsanguinierten Tieren in Phosphat-gepufferter Salzlösung (NaCl: 124 mM; KCl: 5 mM; CaCl: 2 mM; MgSO4: 2 mM; NaH2P04: 1,25 mM; NaHC03: 26 mM: Glucose: 10 mM) KontrollLösung war artifizielle Cerebral-Spinal-FLüsssigkeit (ACSF); Theogallin und Chinasäure wurden von der Firma Plantextrakt, Vestenbergsgreuth, zur Verfügung gestellt. Dann wurde der mittlere Teil des Hippokampus mit Hilfe eines Schnellklebers aufgeblockt und mit einem Vibratom in 400 µm dicke Scheiben geschnitten. Die Aufbewahrung dieser Schnitte erfolgte für mindestens eine Stunde vor Versuchsbeginn (siehe S.J. Schiff, G.G. Somjen: "The effects of temperature on synaptic transmission in hippocampal tissue slices" in Brain Research (1985), 345: 279-284) in einer mit Carbogen durchperlten Inkubationskammer.

Das Experiment selbst wurde in einer sog. "Base Unit" mit "Haas Top" (Firma Medical Systems Corp., USA) bei 35 Grad Celsius durchgeführt. Der mit Hilfe peristaltischer Pumpen (Infusomat B. Braun Melsungen AG) superfundierte Hippokampus-Schnitt lag auf einem Stück Gaze. Eingeleitetes Carbogen hielt die erforderliche Sauerstoffzufuhr der Lösung aufrecht. Die Durchflussgeschwindigkeit betrug 200 ml/h.

Die Stimulation der CA2-Region (Schaffer-Kollateralen) erfolgte unter Verwendung eines Reizgenerators (Laborcomputer Labteam der Fa. MediSyst GmbH, Linden, DE) über eine Isoliereinheit mit Hilfe einer bipolar konzentrischen Stahlelektrode (Rhodes Medical Systems, USA. Die Impulsbreite betrug 200 µs, die Stromstärke konstant 200 µA. Der Reizgenerator löste im Abstand von jeweils 20 Sekunden 4 Einzelreizungen aus, die im Schnitt insgesamt 4 Populationsspikes evozierten. Die Ableitung der Antwort erfolgte in der CA3 Region. Das System mittelte die Reizantwort der 4 Spike-Amplituden.

Die Wirkung der beiden einzelnen Substanzen auf das evozierte Potential wurde nach Einzelreizung wie auch nach Induktion der Langzeitpotenzierung durch einen kurzdauernden (1 s) tetanischen Reiz (90 Hz) untersucht (siehe K.G. Reymann, H.K. Matthies, U. Frey, V.S. Voborbyev, H. Matthies: "Calcium-induced long-term potentiation in the hippocampal slice. Characterization of the time course and conditions" in Brain Bulletin (1986), 17: 291-296). Dabei wurde der Schnitt zunächst mit Kontrolllösung superfundiert. Nach Auffinden eines geeigneten Signals und Registrierung dieses Ausgangsignals während mehrerer Zeitpunkte wurde anstelle der Kontrolllösung auf Theogallin- oder Chinasäure-haltige Lösung umgeschaltet. Jeder Schnitt wurde jeweils nur zu einem Experiment benutzt. Werte werden für n=4 Schnitte angegeben.

Die Ableitung der evozierten Antwort erfolgte in 10-minütigem Abstand extrazellulär mit einer gezogenen Glaskapillare (Elektrodenpuller, Rhema Labortechnik, DE). Das Antwortsignal wurde verstärkt (Verstärker "LMI", List Electronics, Darmstadt, DE), mit einem Laborrechnersystem "Labteam" (Software NeuroTool, MediSyst GmbH, Linden DE) analog digital gewandelt (Auflösung 12 Bit) und ausgewertet. Nach Auffinden eines geeigneten Signals (Amplitudenhöhe etwa 1 mV) wurde die Amplitudenhöhe des Populationsspikes (SAP=Summenaktionspotential; Popspike) als Ausgangsgröße festgelegt. Dieser Referenzwert ergab sich aus dem Mittelwert der letzten drei gemessenen Amplitudenhöhen während Superfusion mit der ACSF Lösung (Ausgangswert). Danach erfolgte die Superfusion mit Testsubstanz mit gleichem Procedere. In Folgeexperimenten erfolgte eine kurzfristige tetanische Reizung (Theta-Burst-Stimulus=TBS) zur Induktion der Langzeitpotenzierung. Hieraus resultierende Amplitudenveränderungen wurden in % dieses Ausgangswertes (n=4) angegeben. Werte in Gegenwart der Testsubstanz sind in % Reduktion dieses Referenzwertes angegeben.

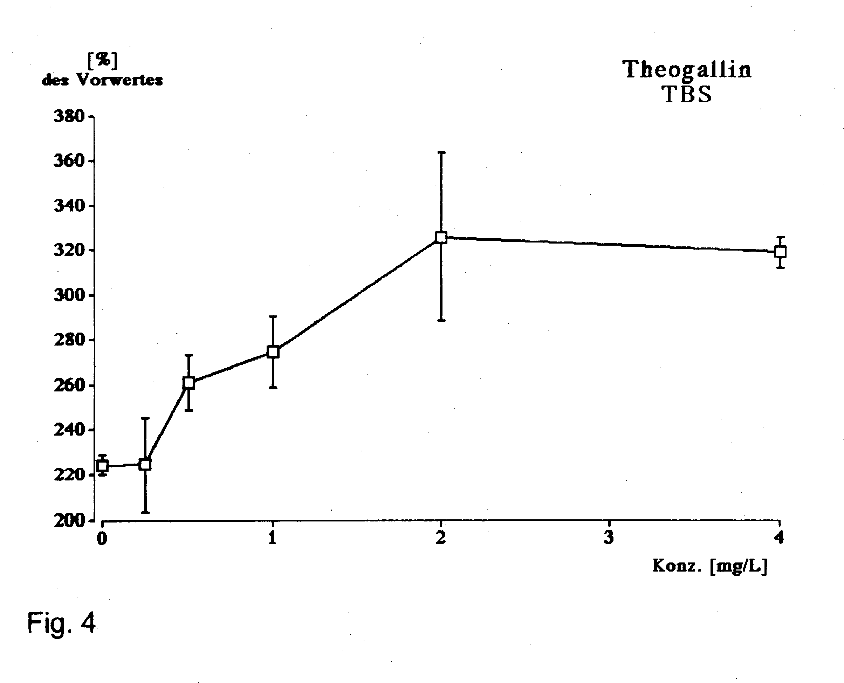

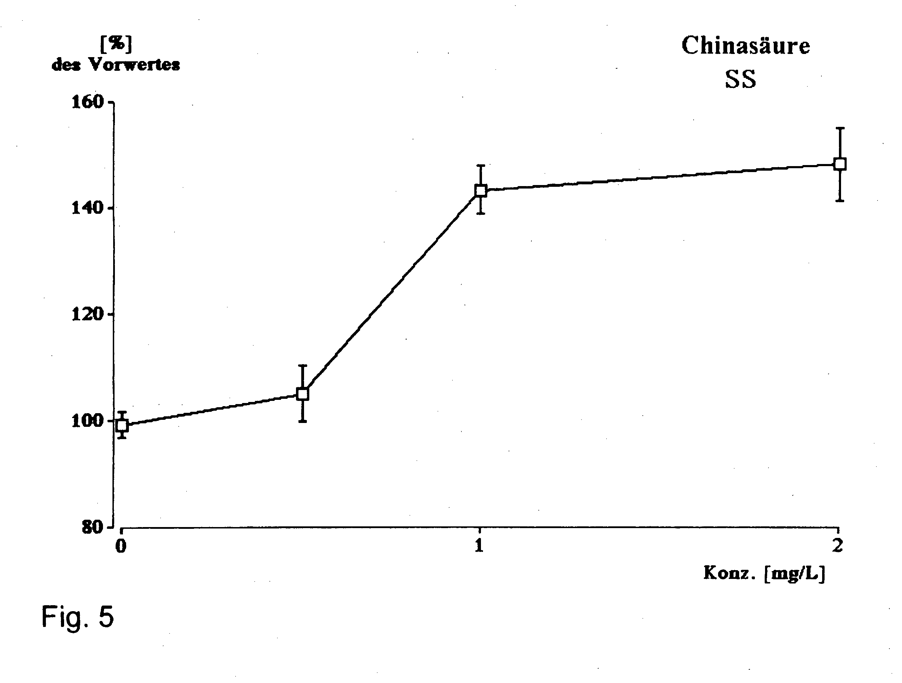

Die Ergebnisse dieser Untersuchungen sind in den Fig. 3 bis 6 gezeigt. Die erste Versuchsanordnung zeigt eine konzentrationsabhängige Zunahme der Amplitude des Populationsspikes in Gegenwart von 0 bis 4 mg/L Theogallin bei Einzelreizung (siehe Fig. 3). Die Auslösung der Langzeitpotenzierung durch TBS wurde bei der gleichen Konzentration konzentrationsabhängig ebenfalls verstärkt (siehe Fig. 4), wie es von Versuchen mit Antidementiva her bekannt ist. Gleichsinnige Ergebnisse wurden in Gegenwart von Chinasäure erhalten (Fig. 5 bis 6). Aus diesem Ergebnis kann geschlossen werden, dass Theogallin und Chinasäure - wie auch aus den EEG-Untersuchungen ersichtlich ist - Depression und Demenzen positiv beeinflussen.

## Patentansprüche

1. Verwendung von Theogallin, seines Metaboliten Chinasäure oder der pharmazeutisch annehmbaren Salze dieser Stoffe zur Herstellung eines Arzneimittels zur Prophylaxe bzw. Behandlung von Demenzen, wie Morbus Alzheimer oder Morbus Parkinson, von Depressionen oder Konzentrationsleistungsstörungen, wie beim Aufmerksamkeitsdefizit-Hyperaktivitätssyndrom.

2. Verwendung nach Anspruch 1, **dadurch gekennzeichnet, dass** das Arzneimittel auf eine Dosierung von 40 bis 300 mg, insbesondere von 80 bis 180, bevorzugt von 120 bis 150 mg pro Tag eingestellt ist.

3. Verwendung nach Anspruch 1 oder 2, **dadurch gekennzeichnet, dass** das Arzneimittel zusätzlich pharmazeutisch annehmbare Träger, Hilfs- und/oder Verdünnungsmittel enthält.

4. Verwendung nach einem der Ansprüche 1,2 oder 3, **dadurch gekennzeichnet, dass** das Arzneimittel zur oralen Verabreichung hergerichtet ist.

## Claims

1. Use of theogallin, its metabolite quinic acid or the pharmaceutically acceptable salts of these substances for the production of a pharmaceutical product for the prophylaxis or treatment of dementia, such as Alzheimer's or Parkinson's disease, depression or concentration disorders, such as attention deficit hyperactivity disorder.

2. Use according to claim 1, **characterised in that** the pharmaceutical product is adjusted for a dosage of 40 to 300 mg, in particular of 80 to 180, preferably of 120 to 150 mg per day.

3. Use according to claim 1 or 2, **characterised in that** the pharmaceutical product also contains pharmaceutically acceptable carriers, auxiliary agents and/or diluting agents.

4. Use according to one of claims 1, 2 or 3, **characterised in that** the pharmaceutical product is prepared for oral administration.

## Revendications

1. Utilisation de théogalline, de son acide quinique, ou des sels pharmaceutiquement acceptables de ces substances, pour la fabrication d'un médicament destiné à la prophylaxie, respectivement au traitement, de démences, comme la maladie d'Alzheimer ou la maladie de Parkinson, de dépressions ou de troubles de la concentration, comme dans le cas du syndrome du déficit d'attention avec hyperactivité.

2. Utilisation selon la revendication 1 **caractérisée en ce que** le médicament est mis au point à une posologie de 40 à 300 mg, en particulier de 80 à 180, de préférence de 120 à 150 mg par jour.

3. Utilisation selon les revendications 1 ou 2 **caractérisée en ce que** le médicament contient complémentairement des véhicules, des produits auxiliaires et/ou des produits diluants pharmaceutiquement acceptables.

4. Utilisation selon l'une des revendications 1, 2 ou 3 **caractérisée en ce que** le médicament est préparé pour une administration orale.
